# EUROPÄISCHE PATENTSCHRIFT

(11) **EP 0 281 547 B1**
(45) Veröffentlichungstag und Bekanntmachung des Hinweises auf die Patenterteilung: **30.06.1993**
(21) Anmeldenummer: 88890039.6
(22) Anmeldetag: 29.02.1988
(51) Int. Cl.: C12M 1/00

(54) **Vorrichtung zur Züchtung von Dauerformen insbesondere filamentöser Mikroorganismen**
Apparatus for the culture of microorganisms in the dormant state, particularly filamentous microorganisms
Appareil pour la culture de micro-organismes en état de dormance, plus particulièrement de micro-organismes filamenteux

(30) Priorität: 05.03.1987 AT 504/87
(43) Veröffentlichungstag der Anmeldung: 07.09.1988
(73) Patentinhaber: VOGELBUSCH GESELLSCHAFT m.b.H., A-1050 Wien (AT)
(72) Erfinder: Kominek, Jiri, Dipl.-Ing., A-1080 Wien (AT); Zehentgruber, Otto, Dipl.-Ing. Dr., A-1160 Wien (AT); Salzbrunn, Wolfgang, Dipl.-Ing. Dr., A-2700 Wiener Neustadt (AT)
(74) Vertreter: Wolfram, Gustav, Dipl.-Ing.

(56) Entgegenhaltungen:
- FR-A- 2 605 016
- GB-A- 926 541
- US-A- 4 111 753
- US-A- 4 262 091

## Beschreibung

Die Erfindung betrifft eine Vorrichtung zur Züchtung von Dauerformen insbesondere filamentöser Mikroorganismen sowie zur Ernte von an der Oberfläche eines Nährmediums gebildeten Dauerformen, mit übereinander in einem kastenförmigen Behälter angeordneten Tassen für das Nährmedium.

Als filamentös werden Mikroorganismen bezeichnet, die ein ausgebreitetes System verzweigter Fäden (Hyphen) bilden, beispielsweise Schimmelpilze und Actinomyceten. Solche Mikroorganismen bilden oft, vor allem wenn sie an der Oberfläche eines flüssigen oder festen Nährmediums wachsen, Dauerformen, welche aus dem Nährmedium herausragen, aus.

Mikrobielle Dauerformen, meist Sporen genannt, werden sehr häufig als Startkultur für Fermentationen verwendet. So werden u.a. Aspergillus niger-Sporen für die Citronensäureproduktion und Sporen der Gattung Trichoderma zur Cellulaseproduktion eingesetzt. Die Verwendung von Sporen bringt den Vorteil mit sich, daß die Mikroorganismen, meist hochgezüchtete Produktionsstämme, über lange Zeit gelagert werden können und ihre Aktivität dabei nicht verlieren.

Für den Gebrauch in Laboratorien und Pilotanlagen genügt die Gewinnung von Sporen aus Kulturen in größeren Petrischalen oder Tassen, wo der Mikroorganismus auf verfestigtem, flüssigem Nährboden wächst und schließlich Sporen ausbildet, die dann entweder trocken mit sterilen Bürsten oder feucht durch Spülen mit einer sterilen physiologischen Kochsalzlösung geerntet werden.

Dieses relativ einfache Verfahren hat jedoch den Nachteil, daß zum einen auf Petrischalen nur wenig Sporen erhalten werden und zum anderen die Luftfeuchtigkeit im Inneren einer Petrischale nur ungenügend kontrolliert werden kann.

Bei der Anzucht auf größeren Tassen müssen einerseits Klimakästen für die Temperierung und andererseits sterile Räume für die Ernte der Sporen vorhanden sein. Außerdem ist die sterile Manipulation mit den Tassen meist sehr umständlich.

Die Regelung der Luftfeuchtigkeit ist von besonderer Wichtigkeit für die Qualität der und die Ausbeute an Sporen. Deshalb wird zur Gewinnung im industriellen Maßstab bisher meist folgende Vorgangsweise gewählt:
Ein Kasten, meist fahrbar, wird mit flachen, übereinander angeordneten und herausnehmbaren Tassen ausgerüstet. Diese Tassen werden mit heißem, noch flüssigem Nährmedium gefüllt und der ganze Kasten wird mit den Tassen in einem Großraumsterilisator sterilisiert. Nach Abkühlen und Verfestigen des Nährbodens wird der Kasten aus dem Sterilisator gefahren und über Sterilfilter an eine Klimaanlage angeschlossen. Danach wird über das Belüftungssystem mit Sporen beimpft, der Mikroorganismus unter zunächst hoher Luftfeuchtigkeit herangezüchtet und nach erfolgter Sporenbildung wird mittels trockener Luft so lange entwässert, bis der Nährboden trocken ist und die Sporen, nach Herausnahme der einzelnen Tassen, in einem sterilen Raum geerntet werden können.

Voraussetzung für die industrielle Produktion war somit bisher die Bereitstellung eines aufwendigen und teuren Großraumsterilisators. Weiters mußten sterile Räume vorhanden sein. Dazu kommt noch, daß die Überführung der Tassen in den sterilen Raum reichlich umständlich und die Ernte der Sporen mit erheblichem Zeitaufwand verbunden war. Für den Fall der Züchtung und Ernte von Sporen pathogener Mikroorganismen war das Personal während der Manipulation außerdem vor des Kontakt mit den Sporen kaum geschützt.

Die Erfindung bezweckt die Überwindung der dargelegten Nachteile und Schwierigkeiten und stellt sich die Aufgabe, eine betriebssichere, mobile, flexibel einsetzbare und kompakte Vorrichtung zu schaffen, welche ausreichend große Produktions- und Manipulationsflächen bietet und nicht vom Vorhandensein eines Großraumsterilisators sowie steriler Räume abhängig ist.

Die gestellte Aufgabe wird bei einer Vorrichtung der eingangs definierten Art erfindungsgemäß dadurch gelöst, daß
- der kastenförmige Behälter mit einer Beschickungskammer und mit einer eine Klimatisierungseinrichtung enthaltenden Klimatisierungskammer in einem gemeinsamen Gehäuse vereinigt ist, wobei zumindest die Frontwand der Beschickungskammer aus durchsichtigem Material gefertigt ist und darin Öffnungen für den Anschluß von Manipulationshilfen, beispielsweise Handschuhen, vorgesehen sind,
- eine gasdicht verschließbare Beschickungsöffnung in einer Seitenwand der Beschickungskammer angeordnet ist,
- sich an der Unterseite der Beschickungskammer ein Anschluß für eine Dauerformen-Ernteeinrichtung befindet,
- die Klimatisierungseinrichtung ein Sterilfilter zur Sterilhaltung von durch den kastenförmigen Behälter und die Beschickungskammer zu leitender Zuluft sowie ein weiteres Sterilfilter zur Filtration der abströmenden Abluft umfaßt, wobei die beiden Sterilfilter über eine in der Klimatisierungskammer befindliche Gasleitung, in welche ein erstes Absperrorgan, eine Kühleinheit, welche über ein zweites Absperrorgan mit einer Kondensatfalle und elektrisch mit einemm Feuchtigkeitsregler, dessen Fühler in den Abschnitt der Gasleitung zwischen einer Heizeinheit und Zuluft-Sterilfilter eingebaut ist, verbunden ist, ein Ventilator und eine Heizeinheit eingebaut sind, miteinander verbunden sind.

Mittels einer solchen Vorrichtung können mehrere Arbeitsschritte - nämlich sterile Anzucht der Mikroorganismen und Induzierung der Bildung von Dauerformen durch regelbare Umweltbedingungen, Trocknung der Dauerformen und sterile Aberntung - ohne hohe Beschaffungskosten unkompliziert und unter durchwegs sterilen Bedingungen ausgeführt werden. Durch die durchsichtige Frontwand kann die Sporenbildung gut beobachtet werden.

Als durchsichtiges Material für die Frontwand der Beschickungskammer kommt beispielsweise Plexiglas in einem Aluminiumrahmen in Frage, wobei die Frontwand zweckmäßig zur Gänze nach oben aufklappbar ausgebildet ist. Diese Ausbildung ist vor allem für den Fall einer von Zeit zu Zeit vorzunehmenden Reinigung sehr günstig, da der Innenraum direkt zugänglich ist.

In den Öffnungen der Frontwand werden Manipulationshilfen luftdicht abschließend befestigt, wobei sich Handschuhe aus Neopren als sehr geeignet erwiesen haben.

Durch die Beschickungsöffnung kann die jeweils benötigte Ausrüstung an Kleingeräten eingebracht werden.

Vorzugsweise weist die obere Abdeckung des gemeinsamen Gehäuses im Bereich des kastenförmigen Behälters und der Beschickungskammer Anschlüsse für Anstechnadeltechnik auf und besteht gegebenenfalls wie die Frontwand aus durchsichtigem Material.

Diese Anschlüsse enthalten ein gegebenenfalls mehrschichtiges Septum aus elastischem Material, beispielsweise Silikongummi. Durch das Septum können sterile Hohlnadeln eingestochen werden, ohne daß unsterile Außenluft in das Gehäuseinnere eindringt. Die Hohlnadeln können über einen Konus mit Spritzenkörpern oder mit einer Leitung verbunden sein, wodurch die Zuführung sterilisierter flüssiger oder gasförmiger Medien in das Gehäuseinnere ermöglicht wird.

Nach einer bevorzugten Ausführungsform ist unterhalb und vor dem kastenförmigen Behälter eine mit Öffnungen versehene, um eine waagrechte Achse schwenkbare Arbeitsplatte vorgesehen, unter der ein Raum zur Aufnahme von Gegenständen angeordnet ist.

Solche Gegenstände können zeitweise nicht benötigte Ausrüstungsstücke oder eventuell anfallende, das weitere Arbeiten behindernde Abfälle sein. Die Gegenstände können auf einfache Weise durch Anheben der Arbeitsplatte bis in annähernd senkrechte Stellung ohne großen Arbeitsaufwand in den darunter befindlichen Raum befördert werden. Die durch den kastenförmigen Behälter, durch die Beschickungskammer und durch den Raum zur Aufnahme von Gegenständen zirkulierende Luft kann ungehindert durch die Öffnungen in der Arbeitsplatte strömen.

Nach einer weiteren vorteilhaften Ausführungsform ist die Dauerformen-Ernteeinrichtung als Zyklon ausgebildet, von dessen seitlichem Anschlußstutzen ein flexibler Schlauch zu dem Anschluß an der Unterseite der Beschickungskammer führt, dessen zentraler Anschlußstutzen über ein Filter mit der Saugseite eines Ventilators verbunden und dessen Austragsrohr gasdicht in einen Sammelkolben eingesetzt ist.

In der Beschickungskammer wird in diesem Fall ein Absaugrohr, welches gleichfalls über einen flexiblen Saugschlauch mit dem Anschluß an der Unterseite der Beschickungskammer verbunden ist, aufbewahrt.

Um die gewünschte Feuchtigkeit der in der erfindungsgemäßen Vorrichtung zirkulierenden Luft einzustellen, wird die Kühlleistung der Kühleinheit und/oder die Verweilzeit in der Kühleinheit, welche beispielsweise aus einem luftgekühlten Kältekompressor und einem Kühlregister bestehen kann, geregelt. Um Vereisung des Kühlregisters zu vermeiden, muß die Temperatur des Kältemittels auf ca. 0°C nach unten begrenzt werden.

Zur genauen Einstellung der gewünschten Zulufttemperatur ist vorteilhaft ein Temperaturfühler in dem Abschnitt der Gasleitung zwischen Heizeinheit und Zuluft-Sterilfilter vorgesehen, welcher Temperaturfühler elektrisch mit einem an die Heizeinheit angeschlossenen Temperaturregler verbunden ist.

Die Heizeinheit kann beispielsweise aus einem oder mehreren elektrischen Heizstäben bestehen. Diese Art der Heizung ist sehr robust und die Heizleistung ist auf einfachste Weise stufenlos verstellbar.

Um auch bestimmte Anteile von Frischluft (Zuluft) beimengen zu können, d.h. ein gewünschtes Verhältnis von Zuluft zu Umluft einstellen zu können und um die Entfernung von gasförmigem Sterilisierungsmittel, wie z.B. Äthylenoxid oder Formaldehyd, aus der erfindungsgemäßen Vorrichtung zu ermöglichen, mündet gemäß einer weiteren bevorzugten Ausgestaltung zwischen der Kühleinheit und dem ersten Absperrorgan eine mit einem dritten Absperrorgan versehene Frischluftleitung in die Gasleitung ein, und zwischen dem ersten Absperrorgan und dem Abluft-Sterilfilter zweigt eine weitere Leitung ab, welche hintereinander mit einem vierten Absperrorgan, einem Hilfsventilator, einem Aktivkohlefilter und einem Luftmengenmesser ausgestattet ist.

Durch diese weitere Leitung kann Gas mittels des Hilfsventilators aus der Vorrichtung abgezogen werden.

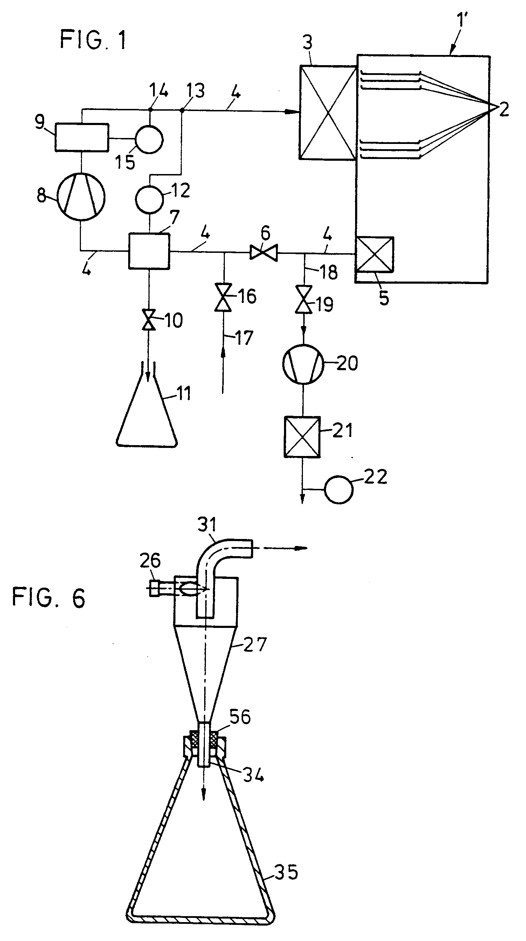

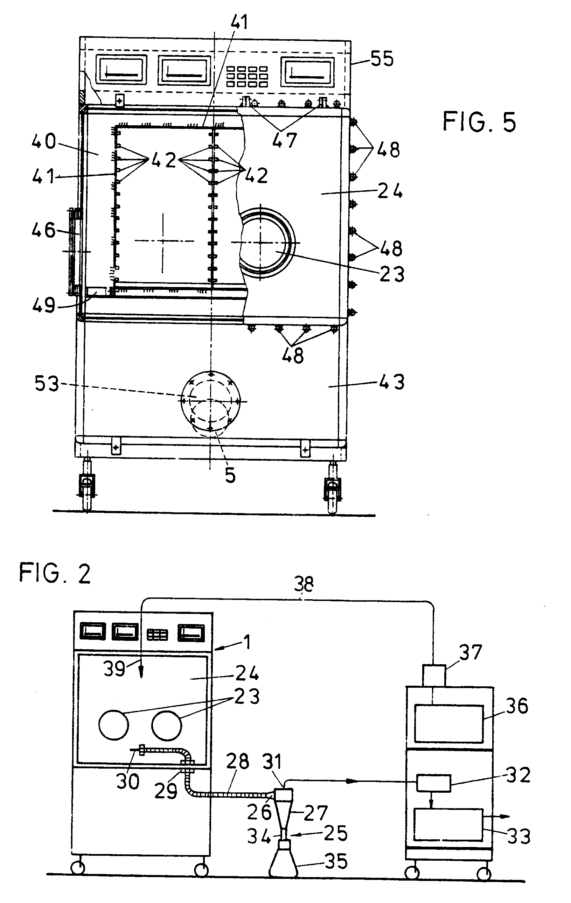

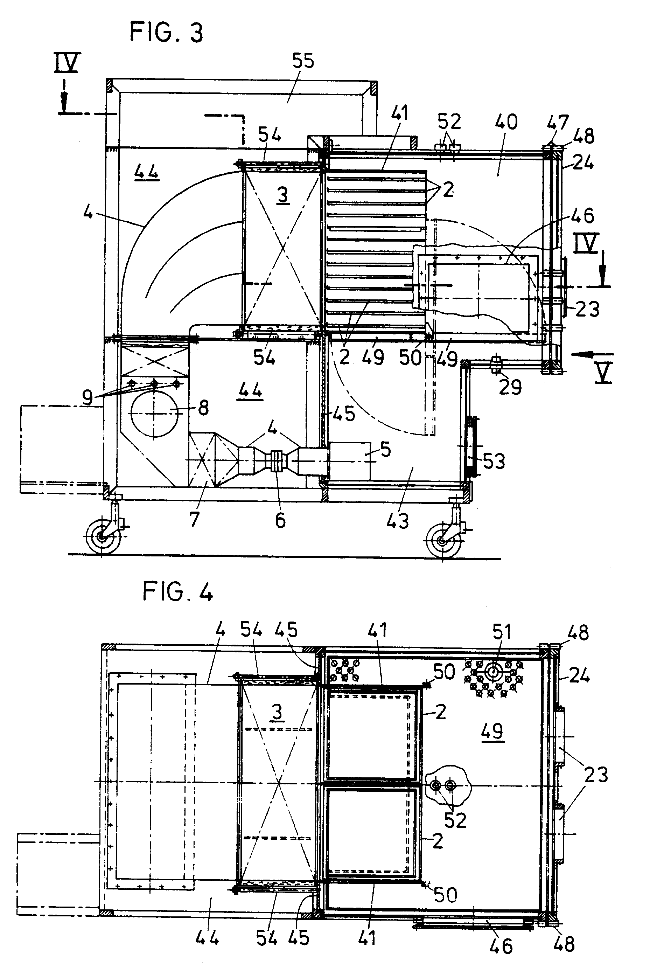

Die Erfindung wird im folgenden anhand der Zeichnung noch näher erläutert. Fig. 1 zeigt eine Prinzipskizze einer Ausführungsform der erfindungsgemäßen Vorrichtung, aus der die Funktionsweise unmittelbar ersichtlich ist. In Fig. 2 ist - gleichfalls schematisch - eine erfindungsgemäße Vorrichtung mit angeschlossener Ernteeinrichtung, Sterilisator für Nährmedium (Agar) und Dosierpumpe für das flüssige, sterilisierte Nährmedium veranschaulicht. Fig. 3 ist eine Darstellung einer speziellen Ausgestaltung einer fahrbaren, erfindungsgemäßen Vorrichtung, großteils im Schnitt, Fig. 4 ist eine Schnittdarstellung der Vorrichtung nach Fig. 3 entlang der Linie IV-IV und Fig. 5 zeigt eine Ansicht der teilweise aufgerissenen Vorrichtung nach Fig. 3 aus Richtung V. Aus Fig. 6 ist eine zweckmäßig mit einer erfindungsgemäßen Vorrichtung kombinierbare Dauerformen-Ernteeinrichtung genauer ersichtlich.

In Fig. 1 ist ein Teil des gemeinsamen Gehäuses - nämlich jener Teil, welcher den kastenförmigen Behälter mit Tassen 2 sowie die Beschickungskammer umschließt - allgemein mit 1ʹ bezeichnet. Ein Sterilfilter 3 zur Sterilhaltung der Zuluft ist direkt an einen nicht eigens dargestellten kastenförmigen Behälter, welcher die Tassen 2 übereinander angeordnet enthält, angebaut. Das Sterilfilter 3 ist über eine Gasleitung 4 mit einem weiteren Sterilfilter 5 zur Filtration der Abluft verbunden. Die Richtung des durch die Gasleitung 4 strömenden Gasgemisches (inbesondere Luft) ist durch einen Pfeil angedeutet. In die Gasleitung 4 sind ein erstes Absperrorgan 6, beispielsweise eine Klappe oder ein Ventil, eine Kühleinheit 7, ein Ventilator 8 und eine Heizeinheit 9 eingebaut.

Die Kühleinheit 7 ist über ein zweites Absperrorgan 10 mit einer Kondensatfalle 11 verbunden. Weiters besteht eine elektrische Verbindung zwischen Kühleinheit 7 und einem Feuchtigkeitsregler 12, welch letzterer wieder elektrisch leitend mit einem entsprechenden - nicht konkret dargestellten - Fühler 13 in dem Abschnitt der Gasleitung 4 zwischen Heizeinheit 9 und Zuluft-Sterilfilter 3 verbunden ist. In dem gleichen Abschnitt der Gasleitung 4 ist weiters ein - gleichfalls nicht konkret dargestellter - Temperaturfühler 14 vorgesehen, welcher elektrisch mit einem Temperaturregler 15 verbunden ist. Der Temperaturregler 15 ist elektrisch auch an die Heizeinheit 9 angeschlossen.

Zwischen dem ersten Absperrorgan 6 und der Kühleinheit 7 mündet eine mit einem dritten Absperrorgan 16 ausgestattete Frischluft(Zuluft-)leitung 17 in die Gasleitung 4 ein und zwischen dem Abluft-Sterilfilter 5 und dem ersten Absperrorgan 6 zweigt eine (Abgas-)Leitung 18 von der Gasleitung 4 ab, in welche hintereinander ein viertes Absperrorgan 19, ein Hilfsventilator 20, ein Aktivkohlefilter 21 und ein Luftmengenmesser 22 eingebaut sind.

Der Fig. 2 läßt sich die Vorderansicht einer fahrbaren erfindungsgemäßen Vorrichtung 1 mit zwei kreisrunden Öffnungen 23 für den Anschluß von Manipulationshilfen in der durchsichtigen Frontwand 24 der Beschickungskammer entnehmen. Oberhalb der Frontwand 24, welche einen Teil des gemeinsamen Gehäuses bildet, ist eine Konsole erkennbar, auf welcher die Skalen der Meßinstrumente sowie Bedienungsknöpfe zur Einstellung der Klimabedingungen im Inneren der erfindungsgemäßen Vorrichtung angeordnet sind.

Eine Dauerformen-Ernteeinrichtung ist allgemein mit 25 bezeichnet, wobei ein seitlicher Anschlußstutzen 26 eines beispielsweise aus Edelstahl gefertigten Zyklons 27 mittels eines flexiblen Schlauches 28 mit einem Anschluß 29 an der Unterseite der Beschickungskammer verbunden ist. Im Inneren der Beschickungskammer führt ein flexibler Schlauch von dem luftdichten Anschluß 29 weiter und endet in einem Absaugrohr 30. Der zentrale Anschlußstutzen 31 des Zyklons 27 ist über ein Filter 32, in welchem restliche, im Zyklon 27 nicht abgetrennte Sporen zurückgehalten werden, mit der Saugseite eines weiteren Ventilators 33 verbunden. Das Austragsrohr 34 des Zyklons 27 ist gasdicht in einen Sammelkolben 35 für Dauerformen eingesetzt.

Aus einem Sterilisator 36 kann heißes, flüssiges Nährmedium mittels einer Dosierpumpe 37 - beispielsweise einer Schlauchquetschpumpe - über die Leitung 38 und eine lediglich schematisch angedeutete Anstechnadel 39 unter sterilen Bedingungen durch die obere Abdeckung des gemeinsamen Gehäuses eingebracht werden.

Es ist zweckmäßig, die erfindungsgemäße Vorrichtung gemeinsam mit den beschriebenen Hilfskomponenten in einem thermostatisierten Raum aufzustellen, wobei die Lufttemperatur des thermostatisierten Raumes immer etwas höher als die Temperatur im Inneren der erfindungsgemäßen Vorrichtung einzustellen ist, um eine Kondensation von Dämpfen an den Wänden des gemeinsamen Gehäuses zu vermeiden.

Zu Beginn eines Züchtungszyklus wird der kastenförmige Behälter mit vorsterilisierten Tassen 2 beschickt und notwendige Geräte werden in die Beschickungskammer eingebracht.

Danach wird der Innenraum der Vorrichtung mit Ausnahme der Klimatisierungskammer durch Verdampfung mit Formaldehydgas gefüllt und einige Stunden zur vollständigen Sterilisation belassen. Äthylenoxid ist wegen erhöhter Explosionsgefahr und der hohen Humantoxizität dieses Gases zur Sterilisation weniger geeignet. Danach wird das erste Absperrorgan 6 geschlossen, das dritte und vierte Absperrorgan 16, 19 werden geöffnet und der Ventilator 8 sowie der Hilfsventilator 20 werden eingeschaltet. Die Formaldehyddämpfe werden abgesaugt und im Aktivkohlefilter 21 kondensiert. Danach wird die Klimatisierungseinrichtung eingeschaltet und Temperatur, Luftfeuchtigkeit und das Frischluft/Umluftverhältnis werden eingestellt. Dazu wird das erste Absperrorgan 6 bei geöffneten dritten und vierten Absperrorganen 16, 19 wieder geöffnet, und durch Regelung der Drehzahl des Hilfsventilators 20 wird das gewünschte - mittels des Luftmengenmessers 22 bestimmbare - Frischluft/Umluftverhältnis eingestellt. Das Nährmedium wird inzwischen im Sterilisator 36 bereitet und mittels der Dosierpumpe 37 über eine Anstechnadel steril in die einzelnen Tassen 2 gepumpt. Bei Verwendung einer Schlauchquetschpumpe wird die jeweils zuzusetzende Menge an Nährmedium zweckmäßig nach vorhergehender Eichung über die Pumpzeit mittels Zeitschaltuhr eingestellt.

Nach Verfestigung des Nährmediums wird eine Impfsuspension der Mikroorganismen bzw. von deren Sporen ebenfalls mittels einer Dosierpumpe oder einer Spritze nach der Anstechnadeltechnik auf die Tassen 2 aufgebracht und beispielsweise mit einer Drigalskispatel verteilt.

Die Züchtungszeit und Sporulationszeit sowie die Züchtungsbedingungen hängen sehr stark vom eingesetzten Mikroorganismus ab. Nach Entstehung der Dauerformen werden die Luftfeuchtigkeit und Temperatur der zirkulierenden Luft auf Trocknungsbedingungen umgestellt.

Nach entsprechender Trocknung werden die gebildeten Sporen mittels der Ernteeinrichtung 25 abgesaugt und auf diese Weise in den Sammelkolben 35, der zweckmäßig aus Glas besteht, transferiert. Danach werden die abgeernteten Tassen aus der Vorrichtung entfernt, die Vorrichtung wird gereinigt und für einen neuen Züchtungszyklus vorbereitet.

Bei der speziellen Ausführungsform einer erfindungsgemäßen Vorrichtung nach den Fig. 3 bis 5 ist der zur Beschickungskammer 40 hin offene kastenförmige Behälter mit 41 bezeichnet. Er enthält Schienen 42 zum Einschieben der Tassen 2, welche das Nährmedium aufnehmen. Die Frontwand 24 der Beschickungskammer 40, die obere Abdeckung des gemeinsamen Gehäuses im Bereich des kastenförmigen Behälters 41 und im Bereich der Beschickungskammer 40, die Unterseite der Beschickungskammer 40, sämtliche Seitenwände dieser Vorrichtungsabteilungen einschließlich aller Begrenzungswände des darunter befindlichen Raumes 43 sind aus Plexiglasscheiben gefertigt, die gasdicht in Aluminiumprofile eingesetzt sind, welch letztere die Kanten des gemeinsamen Gehäuses in diesem Bereich bilden. Die Klimatisierungskammer 44 ist an die gemeinsame Hinterwand 45 des kastenförmigen Behälters 41 und des Raumes 43 angebaut. Die Wände der Klimatisierungskammer 44 bestehen nicht aus durchsichtigem Material.

In der von der Frontwand 24 aus gesehen linken Seitenwand der Beschickungskammer 40 ist eine gasdicht verschließbare rechteckförmige Beschickungsöffnung 46 angeordnet. Die Frontwand 24 ist zur Gänze um Scharniere 47 nach oben aufklappbar. Sie wird im geschlossenen Zustand mittels entlang ihres Randes vorgesehener Befestigungselemente, beispielsweise Schraubverbindungen 48, auf eine rundum verlaufende Dichtung gepreßt. In der Frontwand 24 sind zwei kreisrunde Öffnungen 23 für den luftdichten Anschluß von nicht dargestellten Handschuhen vorgesehen, um in der Beschickungskammer 40 bzw. im kastenförmigen Behälter 41 notwendige Arbeiten unter Aufrechterhaltung steriler Bedingungen vornehmen zu können.

Direkt unterhalb und vor dem kastenförmigen Behälter 41 ist eine mit Öffnungen versehene Arbeitsplatte 49 vorgesehen, welche unter dem kastenförmigen Behälter 41 U-förmig ausgeschnitten und in zwei Lagern 50 schwenkbar gelagert ist. Die in senkrechte Stellung gedrehte Arbeitsplatte ist in Fig. 3 strichpunktiert eingezeichnet. In dieser Stellung der Arbeitsplatte 49 ist auch der darunter befindliche Raum 43, welcher u.a. zur Aufnahme nicht benötigter Ausrüstungsstücke dient, von der Beschickungskammer 40 her zugänglich.

An der Unterseite der Beschickungskammer 40 befindet sich ein Anschluß 29 für die bereits im Zusammenhang mit Fig. 2 beschriebene Dauerformen-Ernteeinrichtung. Zur Durchführung des von dem Anschluß 29 zu einem Absaugrohr 30 führenden flexiblen Schlauches (Fig. 2) ist in der Arbeitsplatte 49 eine Ausnehmung 51 vorgesehen.

In der oberen Abdeckung des gemeinsamen Gehäuses sind weiters zwei Anschlüsse 52 für Anstechnadeltechnik vorgesehen.

Um die Entnahme von Abfällen aus dem Raum 43 zu erleichtern, ist in der Frontwand dieses Raumes eine gleichfalls hermetisch verschließbare Entnahmeöffnung 53 angeordnet. Im Bodenbereich des Raumes 43 ist ein Stück der Gasleitung 4 dichtend durch die gemeinsame Hinterwand 45 in den Raum 43 geführt. Das Ende der Gasleitung ist an ein Abluft-Sterilfilter 5 angeschlossen. Ein Zuluft-Sterilfilter 3 ist mit dem anderen Ende der Gasleitung 4 verbunden. Das Sterilfilter 3 befindet sich in der Klimatisierungskammer 44 und ist über seinen Rahmen 54 dichtend mit dem Rand eines korrespondierenden Ausschnittes in der Hinterwand 45 verbunden. Die über die vergleichsweise große Querschnittsfläche des Sterilfilters 3 direkt hinter den Tassen 2 zugeführte sterile Luft strömt laminar über alle Tassen, wodurch gut regelbare Klimaverhältnisse erreicht werden.

In die Gasleitung 4 sind in Strömungsrichtung der Luft gesehen nach dem Abluft-Sterilfilter 5 hintereinander ein erstes Absperrorgan 6, eine Kühleinheit 7, ein Ventilator 8 und eine drei Elektroheizstäbe umfassende Heizeinheit 9 eingebaut. Die Funktion dieser Vorrichtungsteile wurde bereits im Zusammenhang mit den Fig. 1 und 2 genauer dargelegt.

Die benötigte elektrische bzw. elektronische Ausrüstung, wie die beschriebenen Meß- und Regelgeräte, befinden sich in einem Konsolenraum 55, welcher als Teil der Klimatisierungskammer 44 aufgefaßt werden kann. Außen an der Frontwand des Konsolenraumes 55 befinden sich die Anzeigeskalen der Meßgeräte. Die elektrischen Geräte sowie elektrische Leitungen sind in den Fig. 3 bis 5 nicht dargestellt.

Die Dauerformen-Ernteeinrichtung 25 nach Fig. 2 ist in Fig. 6 vergrößert und teilweise geschnitten dargestellt.

Das Austragsrohr 34 des Zyklons 27 ist mittels eines durchbohrten Stopfens 56 aus elastischem Material gasdicht in den Sammelkolben 35 eingesetzt. Ein mit Sporen beladener Luftstrom gelangt durch den seitlichen Anschlußstutzen 26 tangential in den Zyklon. Der größte Teil der im Luftstrom enthaltenen Sporen wird dabei abgetrennt und fällt durch das Austragsrohr 34 in den Sammelkolben 35. Die nur mehr Restsporen enthaltende Luft verläßt den Zyklon 27 durch den zentralen Anschlußstutzen 31.

Bei einem ungefähren Rauminhalt der Vorrichtung (ohne Klimatisierungskammer) von 0,9 m³ hat es sich als günstig erwiesen, nachstehende Komponenten leistungsmäßig wie folgt auszulegen:
Ventilator 8:
variable Drehzahl, Förderleistung 0 bis 50 m³ Gas (Luft)/h
Hilfsventilator 20:
variable Drehzahl, Förderleistung 0 bis 20 m³ Luft/h
Kühleinheit 7 (Kältekompressor und Kühlregister):
20 m³ Luft/h
33°C auf 4°C
Leistungsaufnahme ca. 700 W
Heizeinheit 9 (drei elektrische Heizstäbe):
Leistung max. 500 W, stufenlos regelbar
Luftmengenmesser 22:
geeignet zum Messen von Abgas- bzw. Frischluft(Zuluft)mengen von 0,5 bis 5 m³/h.

## Patentansprüche

1. Vorrichtung zur Züchtung von Dauerformen insbesondere filamentöser Mikroorganismen sowie zur Ernte von an der Oberfläche eines Nährmediums gebildeten Dauerformen, mit übereinander in einem kastenförmigen Behälter angeordneten Tassen (2) für das Nährmedium, dadurch gekennzeichnet, daß
- der kastenförmige Behälter (41) mit einer Beschickungskammer (40) und mit einer eine Klimatisierungseinrichtung enthaltenden Klimatisierungskammer (44) in einem gemeinsamen Gehäuse vereinigt ist, wobei zumindest die Frontwand (24) der Beschickungskammer (40) aus durchsichtigem Material gefertigt ist und darin Öffnungen (23) für den Anschluß von Manipulationshilfen, beispielsweise Handschuhen, vorgesehen sind,
- eine gasdicht verschließbare Beschickungsöffnung (46) in einer Seitenwand der Beschickungskammer (40) angeordnet ist,
- sich an der Unterseite der Beschickungskammer (40) ein Anschluß (29) für eine Dauerformen-Ernteeinrichtung (25) befindet,
- die Klimatisierungseinrichtung ein Sterilfilter (3) zur Sterilhaltung von durch den kastenförmigen Behälter (41) und die Beschickungskammer (40) zu leitender Zuluft sowie ein weiteres Sterilfilter (5) zur Filtration der abströmenden Abluft umfaßt, wobei die beiden Sterilfilter (3, 5) über eine in der Klimatisierungskammer (44) befindliche Gasleitung (4), in welche ein erstes Absperrorgan (6), eine Kühleinheit (7), welche über ein zweites Absperrorgan (10) mit einer Kondensatfalle (11) und elektrisch mit einemm Feuchtigkeitsregler (12), dessen Fühler (13) in den Abschnitt der Gasleitung (4) zwischen einer Heizeinheit (9) und Zuluft-Sterilfilter (3) eingebaut ist, verbunden ist, ein Ventilator (8) und eine Heizeinheit (9) eingebaut sind, miteinander verbunden sind.

2. Vorrichtung nach Anspruch 1, dadurch gekennzeichnet, daß die Frontwand (24) der Beschickungskammer (40) zur Gänze nach oben aufklappbar ausgebildet ist.

3. Vorrichtung nach wenigstens einem der Ansprüche 1 und 2, dadurch gekennzeichnet, daß die obere Abdeckung des gemeinsamen Gehäuses im Bereich des kastenförmigen Behälters (41) und der Beschickungskammer (40) Anschlüsse (53) für Anstechnadeltechnik aufweist und gegebenenfalls wie die Frontwand (24) aus durchsichtigem Material besteht.

4. Vorrichtung nach wenigstens einem der Ansprüche 1 bis 3, dadurch gekennzeichnet, daß unterhalb und vor dem kastenförmigen Behälter (41) eine mit Öffnungen versehene, um eine waagrechte Achse schwenkbare Arbeitsplatte (49) vorgesehen ist, unter der ein Raum (43) zur Aufnahme von Gegenständen angeordnet ist.

5. Vorrichtung nach wenigstens einem der Ansprüche 1 bis 4, dadurch gekennzeichnet, daß die Ernteeinrichtung als Zyklon (27) ausgebildet ist, von dessen seitlichem Anschlußstutzen (26) ein flexibler Schlauch (28) zu dem Anschluß (29) an der Unterseite der Beschickungskammer (40) führt, dessen zentraler Anschlußstutzen (31) über ein Filter (32) mit der Saugseite eines Ventilators (33) verbunden und dessen Austragsrohr (34) gasdicht in einen Sammelkolben (35) eingesetzt ist.

6. Vorrichtung nach wenigstens einem der Ansprüche 1 bis 5, dadurch gekennzeichnet, daß ein Temperaturfühler (14) in dem Abschnitt der Gasleitung (4) zwischen Heizeinheit (9) und Zuluft-Sterilfilter (3) vorgesehen ist, welcher Temperaturfühler (14) elektrisch mit einem an die Heizeinheit (9) angeschlossenen Temperaturregler (15) verbunden ist.

7. Vorrichtung nach wenigstens einem der Ansprüche 1 bis 6, dadurch gekennzeichnet, daß zwischen der Kühleinheit (7) und dem ersten Absperrorgan (6) eine mit einem dritten Absperrorgan (16) versehene Frischluftleitung (17) in die Gasleitung (4) einmündet und daß zwischen dem ersten Absperrorgan (6) und dem Abluft-Sterilfilter (5) eine weitere Leitung (18) abzweigt, welche hintereinander mit einem vierten Absperrorgan (19), einem Hilfsventilator (20), einem Aktivkohlefilter (21) und einem Luftmengenmesser (22) ausgestattet ist.

## Claims

1. An arrangement for growing permanent forms of, in particular filamentary, microorganisms as well as for harvesting permanent forms formed on the surface of a culture medium, comprising trays (2) for the culture medium superposed in a box-type container, characterised in that
- the box-type container (41) is united in a common housing with a feeding chamber (40) and with an air-conditioning chamber (44) containing an air-conditioning means, wherein at least the front wall (24) of the feeding chamber (40) is made of transparent material and openings (23) are provided therein for connecting manipulation aids, such as gloves,
- a gas-tightly closeable feed opening (46) is provided in a side wall of the feeding chamber (40),
- a connection (29) to a permanent-form harvesting device (25) is disposed on the lower side of the feeding chamber (40),
- the air-conditioning means comprises a sterile filter (3) for keeping sterile the intake air to be conducted through the box-type container (41) and the feeding chamber (40), as well as a further sterile filter (5) for filtering the offstreaming exhaust air, which two sterile filters (3, 5) are connected with each other via a gas duct (4) provided in the air-conditioning chamber (44) and in which a first shut-off organ (6), a refrigerating unit (7) connected, via a second shut-off organ (10), with a condensate trap (11) and, electrically, with a humidity controller (12) whose sensor (13) is incorporated in that section of the gas duct (4) which extends between a heating unit (9) and the intake-air sterile filter (3), a fan (8) and a heating unit (9) are incorporated.

2. An arrangement according to claim 1, characterised in that the front wall (24) of the feeding chamber (40) is designed to be foldable upwards in its entirety.

3. An arrangement according to at least one of claims 1 and 2, characterised in that the upper cover of the common housing includes connections (53) for pricking needle technique in the region of the box-type container (41) and of the feeding chamber (40) and optionally is made of transparent material like the front wall (24).

4. An arrangement according to at least one of claims 1 to 3, characterised in that a bench (49) provided with openings and pivotable about a horizontal axis is arranged below and in front of the box-type container (41), a space (43) being provided therebelow for retaining articles.

5. An arrangement according to at least one of claims 1 to 4, characterised in that the harvesting device is designed as a cyclone (27), from whose lateral connection socket (26) a flexible tube (28) leads to the connection (29) provided on the lower side of the feeding chamber (40), whose central connection socket (31) is connected via a filter (32) to the suction side of a fan (33) and whose exhaust pipe (34) is gas-tightly inserted in a collecting flask (35).

6. An arrangement according to at least one of claims 1 to 5, characterised in that a temperature probe (14) is provided in that section of the gas duct (4) which extends between the heating unit (9) and the intake-air sterile filter (3), which temperature probe (14) is electrically connected with a temperature controller (15) connected to the heating unit (9).

7. An arrangement according to at least one of claims 1 to 6, characterised in that a fresh-air duct (17) provided with a third shut-off organ (16) enters into the gas duct (4) between the refrigerating unit (7) and the first shut-off organ (6), and that a further duct (18) branches off between the first shut-off organ (6) and the exhaust-air sterile filter (5), which duct is consecutively equipped with a fourth shut-off organ (19), an auxiliary fan (20), an activated carbon filter (21) and an air volumeter (22).

## Revendications

1. Dispositif de culture de formes fixes de micro-organismes filamenteux notamment, ainsi que de récolte de formes fixes formées à la surface d'un milieu nutritif, à l'aide de plateaux (2) pour milieu nutritif superposés dans un récipient en forme de caisson, caractérisé en ce que
- le récipient en forme de caisson (41) est réuni à une chambre de chargement (40) et une chambre de climatisation (44) contenant un dispositif de climatisation dans un carter commun, la paroi frontale (24) au moins de la chambre de chargement (40) étant en un matériau transparent et des orifices (23) permettant le raccord d'accessoires de manipulation, par exemple de gants, y étant prévus,
- un orifice de chargement (46) obturable de façon étanche aux gaz est ménagé dans une paroi latérale de la chambre de chargement (40),
- un raccord (29) destiné à un appareillage de récolte de formes fixes (25) se trouve sur la face inférieure de la chambre de chargement (40),
- le dispositif de climatisation comprend un filtre stérile (3) destiné à maintenir stérile l'air à envoyer à travers le récipient en forme de caisson (41) et la chambre de chargement (40) ainsi qu'un autre filtre stérile (5) destiné à la filtration de l'air sortant s'échappant, les deux filtres stériles (3, 5) étant reliés par une conduite de gaz (4) se trouvant dans la chambre de climatisation (44) et dans laquelle sont incorporés un premier organe d'arrêt (6), une unité frigorifique (7) reliée par un second organe d'arrêt (10) à un piège à condensat (11) et électriquement à un hygrostat (12) dont le capteur (13) est incorporé dans le tronçon de la conduite de gaz (4) se trouvant entre l'unité de chauffage (9) et le filtre stérile de l'air entrant (3), un ventilateur (8) et une unité de chauffage (9).

2. Dispositif selon la revendication 1, caractérisé en ce que la paroi frontale (24) de la chambre de chargement (40) est conçue de façon à être relevable vers le haut dans son intégralité.

3. Dispositif selon au moins une des revendications 1 et 2, caractérisé en ce que le couvercle supérieur du carter commun présente des raccords (53) pour la technique de perforation par aiguilles dans la zone du récipient en forme de caisson (41) et de la chambre de chargement (40) et est le cas échéant, comme la paroi frontale (24), en un matériau transparent.

4. Dispositif selon au moins une des revendications 1 à 3, caractérisé en ce qu'il est prévu sous et devant le récipient en forme de caisson (41) un plan de travail (49) muni d'orifices, pouvant pivoter autour d'un axe horizontal, et sous lequel est ménagé un compartiment (43) pour le rangement d'objets.

5. Dispositif selon au moins une des revendications 1 à 4, caractérisé en ce que l'appareillage de récolte est conçu sous la forme d'un cyclone (27) dont le manchon-raccord latéral (26) est relié par un tube souple (28) au raccord (29) se trouvant sur la face inférieure de la chambre de chargement (40) et dont le manchon central (31) est relié par un filtre (32) au côté aspiration d'un ventilateur (33), et dont la conduite d'évacuation (34) est introduite de façon étanche aux gaz dans un ballon collecteur (35).

6. Dispositif introduite selon au moins une des revendications 1 à 5, caractérisé en ce qu'une sonde pyrométrique (14) est prévue dans le tronçon de la conduite de gaz (4) situé entre l'unité de chauffage (9) et le filtre stérile de l'air entrant (3), laquelle sonde pyrométrique (14) est connectée électriquement à un thermostat raccordé à l'unité de chauffage (9).

7. Dispositif selon au moins une des revendications 1 à 6, caractérisé en ce qu'une conduite d'air frais (17) munie d'un troisième organe d'arrêt (16) débouche dans la conduite de gaz (4) entre l'unité frigorifique (7) et le premier organe d'arrêt (6) et qu'une autre conduite (18) dotée successivement d'un quatrième organe d'arrêt (19), d'un ventilateur auxiliaire (20), d'un filtre à charbon actif (21) et d'un débitmètre d'air (22) se détache entre le premier organe d'arrêt (6) et le filtre stérile de l'air sortant (5).
